# EUROPÄISCHE PATENTSCHRIFT

(11) **EP 1 326 995 B1**
(45) Veröffentlichungstag und Bekanntmachung des Hinweises auf die Patenterteilung: **17.01.2007**
(21) Anmeldenummer: 01976273.1
(22) Anmeldetag: 05.10.2001
(51) Int. Cl.: C12P 21/02

(54) **VERFAHREN ZUR SYNTHESE VON PEPTIDEN, PEPTIDMIMETIKA UND PROTEINEN**
METHOD FOR SYNTHESISING PEPTIDES, PEPTIDE MIMETICS AND PROTEINS
PROCEDE DE SYNTHESE DE PEPTIDES, PEPTIDES MIMETIQUES ET PROTEINES

(30) Priorität: 07.10.2000 DE 10049673
(43) Veröffentlichungstag der Anmeldung: 16.07.2003
(73) Patentinhaber: Roche Diagnostics GmbH, 68305 Mannheim (DE); F. HOFFMANN-LA ROCHE AG, 4070 Basel (CH)
(72) Erfinder: BORDUSA, Frank, 06242 Rossbach (DE); JAKUBKE, Hans-Dieter, 01465 Dresden-Langebrueck (DE)
(86) Internationale Anmeldenummer: PCT/EP2001/011500
(87) Internationale Veröffentlichungsnummer: WO 2002/031177

(56) Entgegenhaltungen:
- WO-A-92/02615
- WO-A-94/18329
- XU S ET AL: "Enzymatic coupling of specific peptides at nonspecific ligation sites: effect of Asp189Glu mutation in trypsin on substrate mimetic-mediated reactions." JOURNAL OF ORGANIC CHEMISTRY, (2001 MAR 9) 66 (5) 1627-32. JOURNAL CODE: 2985193R. , 25. Januar 2001 (2001-01-25), XP002218036
- GRUNBERG R ET AL: "Peptide bond formation mediated by substrate mimetics. Structure-guidedoptimization of trypsin for synthesis." EUROPEAN JOURNAL OF BIOCHEMISTRY, (2000 DEC) 267 (24) 7024-30. JOURNAL CODE: 0107600. , XP002218037
- CEROVSKY V ET AL: "PROTEASE-CATALYZED FRAGMENT CONDENSATION VIA SUBSTRATE MIMETIC STRATEGY: A USEFUL COMBINATION OF SOLID-PHASE PEPTIDE SYNTHESIS WITH ENZYMATIC METHODS" JOURNAL OF PEPTIDE RESEARCH, MUNKSGAARD INTERNATIONAL PUBLISHERS, COPENHAGEN, DK, Bd. 55, Nr. 4, April 2000 (2000-04), Seiten 325-329, XP000930485 ISSN: 1397-002X
- CEROVSKY V ET AL: "Semisynthesis of Ht31(493--515): involvement of PKA-anchoring proteins in the regulation of the cAMP-dependent chloride current in heart cells." CHEMBIOCHEM: A EUROPEAN JOURNAL OF CHEMICAL BIOLOGY. GERMANY 18 AUG 2000, Bd. 1, Nr. 2, 18. August 2000 (2000-08-18), Seiten 126-129, XP002218038 ISSN: 1439-4227
- BORDUSA F: "Nonconventional amide bond formation catalysis: programming enzyme specificity with substrate mimetics." BRAZILIAN JOURNAL OF MEDICAL AND BIOLOGICAL RESEARCH = REVISTA BRASILEIRA DE PESQUISAS MEDICAS E BIOLOGICAS / SOCIEDADE BRASILEIRA DE BIOFISICA... [ET AL.]. BRAZIL MAY 2000, Bd. 33, Nr. 5, Mai 2000 (2000-05), Seiten 469-485, XP008009710 ISSN: 0100-879X
- JAKUBKE H D (REPRINT): "PEPTIDE LIGASES - TOOLS FOR PEPTIDE-SYNTHESIS" ANGEWANDTE CHEMIE-INTERNATIONAL EDITION IN ENGLISH, (03 FEB 1995) VOL. 34, NO. 2, PP. 175-177. , XP002218039
- KURTH T ET AL: "Engineering the S1' subsite of trypsin: design of a protease which cleaves between dibasic residues." BIOCHEMISTRY, (1998 AUG 18) 37 (33) 11434-40. JOURNAL CODE: 0370623. , XP002218040
- GRÁF ET AL.: "Electrostatic complementarity within the substrate-binding pocket of trypsin" PROC. NATL. ACAD. SCI., Bd. 85, Juli 1988 (1988-07), Seiten 4961-4965, US
- EVNIN ET AL.: "Substrate specificity of trypsin investigated by using a genetic selection system" PROC. NATL. ACAD. SCI., Bd. 87, September 1990 (1990-09), Seiten 6659-6663, US

## Beschreibung

Die Erfindung betrifft ein Verfahren zur Herstellung von Peptiden, Peptidmimetika und Proteinen durch Verwendung eines Enzyms, dessen Spezifität dergestalt verändert wurde, dass es der Struktur der Esterabgangsgruppe angepaßt wurde.

Die Synthese von Peptiden, Peptidmimetika und Proteinen besitzt zunehmende Bedeutung für das systematische Studium von Struktur-Funktions-Beziehungen der Proteine als funktionelle Genprodukte und trägt entscheidend zur Entdeckung neuer wirksamer Therapeutika bei (vgl. H.-D. Jakubke, *Peptide: Chemie und Biologie,* Spektrum Akademischer Verlag, Heidelberg, Berlin, Oxford, 1996). Das Design von Peptidligasen (H.-D. Jakubke, *Angew. Chem.* **1995**, 107, 189), d.h. von Enzymen, die in der Lage sind, die Knüpfung einer spezifischen Peptidbindung zwischen zwei Peptidsegmenten selektiv und irreversibel zu katalysieren, stellt eine große Herausforderung dar, da im Laufe der Evolution von der Natur ein solches Enzym bisher nicht entwickelt werden konnte. Eine entscheidende Ursache dafür ist der nicht verifizierbare, ungemein hohe Spezifitätsanspruch, da eine solche universelle CN-Ligase alle möglichen Kombinationen der 21 proteinogenen Aminosäuren, wie sie in C- und N-terminaler Position der zu verknüpfenden Peptidsegmente statistisch vorkommen könnten, molekular erkennen müßte, um eine selektive, irreversible Ligation zu katalysieren. Von der Natur wurde daher für die Proteinsynthese in der Zelle ein schrittweiser Aufbau vom N- zum C-Terminus unter der Katalyse der Peptidyltransferase, die mit hoher Wahrscheinlichkeit ein evolutiv sehr altes Ribozym (B. Zhang, T.R. Cech, *Nature* **1997**, 390, 96) ist, gewählt, wobei die Spezifitätserkennung der zu verknüpfenden Aminosäuren hauptsächlich durch Codon-Anticodon-Wechselwirkung sowie durch eine vorgelagerte Katalyse der hochspezifischen Aminoacyl-tRNA-Synthetasen erfolgt.

Die Nutzung des reversen Katalysepotentials von Peptidasen (vgl. u.a. W. Kullmann, *Enzymatic Peptide Synthesis,* CRC Press, Boca Raton, **1987**; H.-D. Jakubke, *Enzymatic Peptide Synthesis,* in: *The Peptides: Analysis, Synthesis, Biology,* Vol. 9, (Eds.: S. Udenfriend, J. Meienhofer), Academic Press, New York, **1987**, Chapter 3) bietet zwar die prinzipielle Möglichkeit, Peptidsegmente unter speziellen Voraussetzungen enzymatisch zu verknüpfen, doch ist weder die lrreversibilität der geknüpften speziellen Peptidbindung garantiert noch sind unerwünschte proteolytische Spaltungen in den zu verknüpfenden Segmenten bzw. im Endprodukt a priori auszuschließen, wenn sich dort potentielle Spaltstellen für die eingesetzte Peptidase befinden. Obgleich durch Reenginering verschiedener Peptidasen, wie z.B. Subtilisin das Katalysepotential zur Peptidbindungsknüpfung verbessert und auch durch anspruchsvolle Fragmentkondensationen (vgl. u.a. D.Y. Jackson et al., *Science* **1994,** 266, 243) belegt werden konnte, können dadurch die oben geschilderten Nachteile nicht ausgeräumt werden.

Auch katalytische Antikörper (vgl. u.a. P.G. Schultz, R.A. Lerner, *Science* **1995**, 269, 1835; G. MacBeath, D. Hilvert, *Chem. Biol.* **1996**, 3, 433; D.B. Smithrub et al., J. *Am. Chem. Soc.* **1997**, 119, 278) zeigen CN-Ligaseaktivität, ebenso wie synthetische Peptidligasen basierend auf einem coiled-coil-Motif von GCN4 (K. Severin et al., *Nature* **1997**, 389, 706) bzw. auf einer Peptidmatrize bestehend aus einem stark saurem *coiled*-*coil*-Peptid (S. Yao, J. Chmielewski, *Biopolymers* **1999**, 51, 370), wobei es sich in allen diesen Fällen um interessante Ansatzpunkte für das Design von Peptidligasen handelt, die für Ligationen spezielle Voraussetzungen erfordern und deren allgemeine Anwendbarkeit daher äußerst stark limitiert ist.

Die basierend auf dem schon vor vielen Jahren vorgeschlagenen Konzept der Molekülklammer für chemische CN-Ligationen (T. Wieland et al., *Annalen* **1953**, 583, 129; M. Brenner et al., *Helv. Chim. Acta* **1957**, 40, 1497) entwickelten Methoden des Amin- und Thioleinfangs (D.S. Kemp et al., J. *Org. Chem.* **1975**, 40, 3465; N. Fotouhi et al., *J. Org. Chem.* **1989**, 54, 2803), der natürlichen chemischen Ligation (M. Schnölzer, S.B.H. Kent, *Science* 1992, 256,221; P.E. Dawson et al., *Science* **1994,** 266, 776) oder auch der Aldehyd-Methode (C.-F. Liu, J.P. Tam, *Proc. Natl. Acad. Sci. USA* **1994**, 91, 6584) erfordern für deren Realisierung ganz bestimmte N- bzw. C-terminale Aminosäurereste, so dass die Anwendbarkeit sequenzspezifische Voraussetzungen erfordert. Bei der nativen chemischen Ligation erfolgt die Verknüpfung eines synthetischen Peptids mit einer C-terminalen Thioestergruppierung mit einem zweiten Peptid oder Protein, das einen N-terminalen Cysteinrest enthalten muß. Unter Nutzung der Erkenntnisse des Protein-Spleißens (vgl. Review: C.J. Noren et al., *Angew. Chem.* **2000**, 112, 458) wurde die native chemische Ligation zur Inteinvermittelten Protein-Ligation *(expressed protein ligation,* EPL) weiterentwickelt (T.W. Muir et al., *Proc. Natl. Acad. Sci. USA* **1998**, 95, 6705; G.J. Cotton et al., *J. Am. Chem. Soc.* **1999**, 121, 1100), wobei die Thioestergruppierung der Carboxykomponente aus einem rekombinanten Protein, das mit einem spaltungskompetenten Intein fusioniert ist, durch thiolytische Spaltung auf einer Säule gebildet wird. Der Nachteil der Notwendigkeit eines N-terminalen Cysteinrestes am N-Terminus der Aminokomponente für die Ligation konnte natürlich dadurch nicht behoben werden. Der sich bei der thiolytischen Spaltung auf der Säule mit 2-Mercaptoethansulfonsäure bzw. Thiophenol bildende Thioester kann zwangsläufig nicht nur erst nach der Umesterung, sondern auch direkt von der terminalen α-Aminogruppe der zugesetzten Aminokomponente nukleophil angegriffen werden, so dass eine partielle Epimerisierung des C-terminalen Aminosäurerestes nicht ausgeschlossen werden kann.

Aufgabe der Erfindung ist das rationale und evolutive Design von Enzymvarianten mit einer gezielt an Substratmimetikastrukturen angepaßten Spezifität bei gleichzeitig signifikant verminderter proteolytischer Aktivität gegenüber Peptiden bzw. Proteinen.

Diese Aufgabe wird gelöst mit einem Verfahren zur Herstellung von Peptiden, Peptidmimetika und Proteinen, wobei die Spezifität eines im Syntheseprozess als Biokatalysator verwendeten Enzyms dergestelt verändert wird, dass es der Struktur der Esterabgangsgruppe angepaßt wird, was durch eine chemische oder rationale oder evolutive gentechnische Veränderung des Enzyms bewirkt wird, die sich auf die spezifitätsbestimmenden oder katalytisch bedeutsamen Aminosäurereste des Enzyms in der Weise richtet, dass die entstandene künstliche C-N-Ligase die Spezifitätsdetermimante in der Abgangsgruppe des Substratmimetikums als molekulare und katalytisch bedeutsame Region erkennt und dergestalt Bindungen zwischen den Aminosäurebausteinen geknüpft werden.

Gemäß der vorliegenden Erfindung erfolgt das Design künstlicher CN-Ligasen entgegen der vorherrschenden Meinung der Fachwelt in der Weise, dass die Spezifität eines Enzyms der Struktur der Esterabgangsgruppe angepaßt wird. Dies kann sowohl durch eine chemische wie auch rationale bzw. evolutive gentechnische Veränderung des Enzyms erfolgen, wobei diese sowohl die spezifitätsbestimmenden Enzymbindungsregionen als auch primär nur wenig spezifische Bindungsbereiche bzw. katalytisch bedeutsame Aminosäurereste des Enzyms umfassen kann. Die Spezifitätsdeterminante in der Abgangsgruppe des Substratmimetikums bildet nunmehr die molekulare und katalytisch produktive Erkennungsregion für die künstliche CN-Ligase, so dass Bindungen zwischen den Aminosäurebausteinen nicht mehr gespalten, wohl aber geknüpft werden. Die Begriffe Abgangsgruppe sowie Spezifitätsdeterminante sind dem Fachmann bekannt (F. Bordusa, Braz.J.Med.BioLRes. 72 (2000) 469-485).

Da nach der auf dem erfindungsgemäßen Wege erfolgten biokatalytischen Knüpfung der CN-Bindung die eingesetzte Peptidase aufgrund der im Syntheseprodukt nicht mehr vorhandenen spezifischen Esterabgangsgruppe die synthetisierte, wie auch alle anderen CN-Bindungen nicht mehr als Substrat erkennt und somit enzymatische Spaltungen ausgeschlossen werden, sind die erfindungsgemäßen Ergebnisse sehr überraschend.

Vorzugsweise werden für das erfindungsgemäße Design Peptidase-Varianten aber auch Vertreter anderer Enzymklassen bzw. -subklassen wie z.B. Esterasen eingesetzt.

Die erhaltenen Peptide und Proteine können mit üblichen Methoden der Peptid- und Proteinchemie separiert und gereinigt werden.

Proteolytisch inaktive Peptidligasen, insbesondere Peptidase-Varianten und beispielsweise Esterase-Varianten, als Bioakatalysator für die Substratmimetikavermittelte Peptidsynthese lassen sich gentechnisch insbesondere mittels dreier Strategien aus nativen Proteasen erzeugen. Dies ist zum einen die direkte Manipulation der Substratspezifität, deren vorrangiges Ziel die Erzeugung auf das Substratmimetikum angepasster Enzymspezifitäten ist. Dies sollte zwangsläufig immer dann zu einer Minimierung der nativen Spezifität führen, wenn sich die Struktur des Substratmimetikums von der Struktur der spezifischen Aminosäure (Arg und Lys im Falle von Trypsin) unterscheidet. Unter diesem Gesichtspunkt ist z.B. der Austausch von Asp189 gegen basische Reste besonders erfolgversprechend. Seit längerem ist bekannt, dass Asp189 die Arg/Lys-Spezifität des Enzyms bewirkt und somit den für die native Spezifität des Trypsins bedeutsamsten Aminosäurerest darstellt. (L. Graf et al., Proc. Natl. Acad. Sci. USA, 1988 85, 4961; L.B. Evnin et al., Proc. Natl. Acad. Sci. USA, 1990 87, 6659). Der Austausch von Asp189 gegen His, Lys und Arg führt somit zu einer artifiziellen Spezifität des Trypsins für aromatische oder auch aliphatische Strukturen mit einer negativen Carboxylat-Gruppierung, wie beispielsweise 4-Carboxyphenyl-, 4-Carboxybenzyl-, 3-Carboxyethyl-Ester oder auch Aminosäureester der 5-Hydroxy-indol-2-carbonsäure, um nur einige geeignete Substratmimetika-Typen für diese Peptidligasen zu nennen. Aminosäureseitenketten proteinogener Aminosäuren werden hingegen kaum noch produktiv in die modifizierte Bindungstasche eingepasst. Folglich ist im Gegensatz zum Wildtyp-Enzym die Geschwindigkeit der Proteolyse deutlich verlangsamt bzw. vernachlässigbar. Darüber hinaus führt die Kombination dieser Mutationen mit zusätzlichen Mutationen im S'-Bereich des Trypsins (Enzymbereich C-terminal zur Spalt- bzw. Ligationsstelle) zu proteolytisch inaktiven Peptidligasen und zwar immer dann, wenn diese Mutation die Spezifität für die Abgangsgruppe des Substratmimetikums erhöht. So bewirkt die Mutation K60E der Trypsin-Doppelmutante D189K,K60E eine Verschiebung der Einbindung der 4-Guanidinophenylester aus der S1- in die S1'-Bindungsstelle des Enzyms. Auch für die bisher spaltungssensitiven Aminosäuren Lys und Arg ist eine derartige Verschiebung festzustellen. Während letztere jedoch zu einer unproduktiven Einbindung führt, die keine Spaltung zur Folge hat, wird das Substratmimetikum produktiv eingepasst und führt zur Synthese.

Neben der direkten Manipulation der Enzymspezifität ist die Substitution von Aminosäuren der katalytischen Triade des Enzyms (im Fall von Trypsin His75, Asp102 und Ser195) möglich. Bekannt ist, dass für die Spaltung von Peptidbindungen im Gegensatz zu (aktivierteren) Esterbindungen oder aktivierten Amidbindungen wie z.B. in Peptid-4-Nitroaniliden neben der erhöhten Nukleophilie der OH-Funktion des aktiven Ser195 vor allem auch die Protonierung der Aminofunktion der gespaltenen Bindung essentiell ist. (A. Fersht, Enzyme Structure & Mechanism, 2^{nd} ed., W.H. Freeman & Co., New York, 1984). Somit führt der Austausch von Asp102 und/oder His57 zu einer Diskriminierung des Enzyms zwischen Esterase- und Amidaseaktivität auch bei Substratmimetika-vermittelten Reaktionen. Ein ähnlicher Effekt auf die proteolytische Aktivität wäre auch für den Austausch des aktiven Ser195 gegen Thr oder Cys denkbar.

Die dritte Strategie schließlich umfasst Studien zur indirekten Manipulation der Enzymaktivität mit dem Ziel einer Stabilisierung Zymogen-ähnlicher und damit proteolytisch inaktiver Enzymkonformationen (Zymogene sind die natürlich vorkommenden Vorläuferenzyme von Proteasen, die allerdings proteolytisch inaktiv sind). Während für das "echte" Zymogen, Trypsinogen Lys15Gly (Spaltung der Lys15-Ile16-Bindung führt zur Umwandlung von Trypsinogen zum aktiven Trypsin) keine Peptidligase-Aktivität nachzuweisen ist, besitzen Zymogen-ähnliche Enzymspezies durchaus Peptidligase-Charakter. Zymogen-ähnliche Enzymspezies sind z.B. die Enzymvariante Asp194Asn (Asp194 stabilisiert die aktive Enzymkonformation durch Ausbildung einer Salzbrücke zum N-Terminus des aktivierten Trypsins) wie auch die Variante Cys191Ala (Cys191 stabilisiert das aktive Enzym durch die Bildung einer Disulfidbrücke).

Die vorliegende Erfindung betrifft weiterhin ein Verfahren zur Herstellung von im wesentlichen proteolytisch inaktiven Peptidligasen, insbesondere Trypsinvarianten mittels gentechnologischer Methoden. Dabei wird die gewünschte Mutation ausgehend von Vektoren mit der Protease-codierenden Sequenz, beispielsweise mit Trypsin- oder Trypsinogen-codierender Sequenz, eingeführt. Beispielsweise kann der E. coli Vektor pST verwendet werden, ein Hefe Shuttlevektor mit ADH/GAPDH-Promotor und einer α-Faktor Leadersequenz, welche mit der Trypsinogen-codierenden Sequenz fusioniert ist (L. Hedstorm et al., Science 255 (1992) 1249). Danach erfolgt die Transformation des neuen Vektors in einem geeigneten Wirt, beispielsweise in E. coli. Falls notwendig kann die modifizierte Protease-Sequenz beispielsweise die modifizierte Trypsin- oder Trypsinogensequenz in geeigneten Expressionsvektoren beispielsweise dem Hefevektor pYT subkloniert werden. Falls die Mutation direkt von einem Expressionsvektor ausgeht, ist dieser zusätzliche Schritt hinfällig. Danach erfolgt die Expression der Protease-Variante in der Wirtszelle beispielsweise in E. coli oder Hefe. Die Isolierung der modfizierten Protease-Variante beispielsweise des modifizierten Trypsins bzsw. seines Zymogens, erfolgt mit geeigneten proteinbiochemischen Methoden wie beispielsweise lonenaustauschchromatographie. Falls es sich bei der Protease-Variante um ein Zymogen z.B. Trypsinogen-Variante handelt, erfolgt in üblicher Weise die Aktivierung durch Überführung der Zymogen-Variante in die Protease-Variante, beispielsweise durch proteolytische Spaltung. Falls notwendig können noch weitere Aufreinigungsschritte, z.B. mittels Affinitätschromatographie (falls nach der Modifizierung eine Bindung an SBTJ noch möglich ist) oder mittels Perfusionschromatographie erfolgen. Letztendlich wird falls notwendig die Protease-Variante gegen einen geeigneten Puffer dialysiert und aufkonzentriert.

Die vorliegende Erfindung wird vom Prinzip her am Beispiel der Trypsinvariante D189K,K60E sowie deren Anwendung als CN-Ligase bei einer Segmentkondensation erläutert. Bei dieser Trypsinmutante ist die Aminosäure D an Position 189 durch K und die Aminosäure K an Position 60 durch E ersetzt. Die Sequenz des Trypsin-Wildtyps ist dem Fachmann bekannt. Die Trypsinspezies D189K,K60E wurde speziell für die Erkennung der 4-Guanidinophenylester-Abgangsgruppe entwickelt. Aufgrund der Ladungsumkehr in der primär spezifitätsbestimmenden S1-Bindungstasche durch die Mutation D189K wird die Einbindung der ursprünglich für Trypsin spezifischen Aminosäurereste Lys und Arg verhindert. Diese Mutation allein führt allerdings auch zu einer signifikanten Verringerung der Aktivität gegenüber den Acyl-4-Guanidinophenylester-Substratmimetika. Der zusätzliche Austausch von Lys 60 gegen Glu bewirkt erfindungsgemäß und überraschend für die Fachwelt die Ausbildung einer neuen, für die 4-Guanidinophenylester-Abgangsgruppe spezifischen Bindungstasche in der S1 `-Bindungsortregion des Enzyms. Die Folge ist eine signifikante Erhöhung der Spezifität gegenüber Acyl-4-Guanidinophenylestern, während dessen die Unspezifität gegenüber den ursprünglich spezifischen Aminosäureresten erhalten bleibt. Die Trypsinvariante D189K,K60E findet Verwendung bei der Herstellung von Peptiden, Peptidmimetika und Proteinen. Die Wahl des Puffersystems, der Reaktionszeit und anderer Parameter, wie beispielsweise Reaktionstemperatur etc. ist verhältnismäßig unkritisch und kann von einem Fachmann für enzymatische Transformationen einfach ermittelt werden.

Bei der Herstellung von Peptiden, Peptidmimetika und Proteinen wird als Carboxylkomponente ein Acyl-4-Guanidinophenylester beispielsweise ein Peptid-4-Guanidinophenylester und als Aminokomponente ein weiteres Peptid oder Peptidmimetika eingesetzt. Durch die Ligation der Aminokomponente und der Carboxylkomponente entsteht das gewünschte vollständige Peptid, Peptidmimetikum oder Protein. Überraschenderweise führt diese Art der Synthese weder zu einer Modifizierung von funktionellen Aminosäureseitenketten von trifunktionellen Aminosäuren, wie beispielsweise Lysin, noch zu einer detektierbaren proteolytischen Spaltung der Reaktanden bzw. des Ligationsproduktes.

Die im folgenden beschriebene Synthese des biologisch aktiven Ht31 (493-515) Peptides bestätigt den darauf basierenden irreversiblen Ligase-Charakter dieser Substratmimetika-spezifischen Trypsin-Variante.

### Beispiel 1 - Herstellung der Trypsinmutante D189K, K60E

### Plasmide

Zur Durchführung der ortsgerichteten Mutagenesen kam der *E*. *coli* Vector pST zum Einsatz. Dieser enthält einen Teil des Bluescript-Vectors sowie das Gen für anionisches Rattentrypsin, das mit einem α-factor-leader sowie einem ADH/GAPDH-Promotor fusioniert ist.

Die Proteinexpression erfolgte mit Hilfe des pYT-Plasmides, einem pBS24-Abkömmling, das Selektions-Marker für Uracil- und Leucin-defizientes Medium trägt.

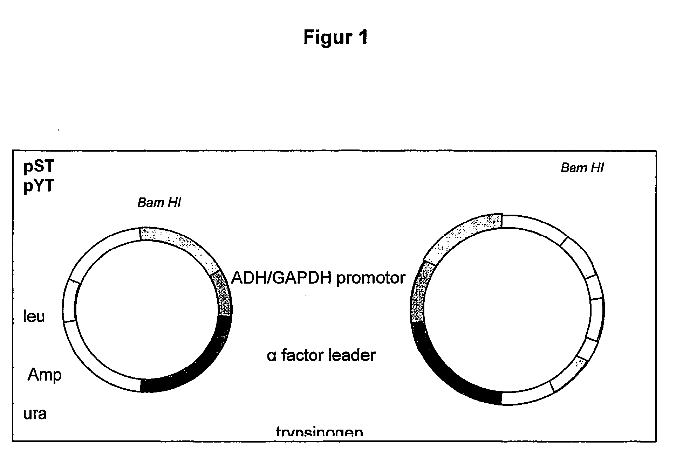

Sowohl das pST- als auch das pYT- Plasmid verfügt über ein Ampicillinresistenz-Gen. Die Karten beider Vektoren, d.h. der Plasmide pST (5,4 kb)- und pYT (14 kb), mit den entsprechenden Schnittstellen sind in Figur 1 dargestellt.

### Mutagenese

Die ortsgerichteten Mutagenesen wurden unter Verwendung des Quik change ^{®} -Kit (STRATAGENE) im *E*. *coli* Plasmid pST durchgeführt.

Das verwendete Verfahren ähnelt einer PCR, wobei ausgehend von zwei synthetischen Oligonukleotidprimern, welche die gewünschte Mutation enthalten, beide Plasmidstränge des pST-Vectors von PFU-Polymerase repliziert werden. Wildtyp pST diente als Template zur Erzeugung von einzelnen Mutationen. Diese Einzelmutanten waren wiederum Ausgangspunkt für die Konstruktion der Doppel-Mutante.

Zum Einsatz kamen folgende Oligonukleotid-Primer, wobei die fettgedruckten Buchstaben die Mutationen angeben:
- D189K: **a)** 5' - GGA GGC AAG **AAC** GAT TCC TGC - 3'
**b)** 5' - GCA GGA ATC **GTT** CTT GCC TCC - 3'
- K60E: **a)** 5' - CAC TGC TAT **GAG** TCC CGC ATC - 3'
**b)** 5' - GAT GCG GGA **CTC** ATA GCA GTG - 3'

Das erhaltene PCR-Produkt wurde in ultrakompetente *E*. *coli* XL II blue Zellen (STRATAGENE) transformiert. Die anschließende Selektion erfolgte auf Ampicillinhaltigen Nähragarplatten (LB-amp). Die gepickten Kolonien wurden in ein Ampicillinhaltiges Flüssigmedium (LB-amp) überführt wobei nach eintägiger Kultivierung die Isolierung des Plasmides unter Verwendung des SNAP-Kit (INVITROGENE) durchgeführt wurde. Die Kontrolle der isolierten DNA erfolgte mittels Elektrophorese mit einem 1 %-igen Agarose-Gel. Durch Sequenzierung des kompletten Gens konnte sichergestellt werden, dass nur die gewünschten Mutationen enthalten waren.

### Subklonierung

Für alle Mutanten die im pST-Plasmid erzeugt wurden, war eine Subklonierung im pYT-Expressions-Vektor notwendig. Diese erfolgte durch Restriktionsverdau mit Bam HI und Sal I und Ligation in das korrespondierende pYT-Vektorfragment. Alle Vektorfragmente wurden im entsprechenden Restriktionsmix auf ein niedrig schmelzendes Agarose-Gel (0,8%) aufgetragen und nach ausreichender Auftrennung ausgeschnitten. Die Gelstücke wurden bei 55 °C geschmolzen und nach gewünschter Kombination vereinigt und bei 16 °C über Nacht mit T4 DNA-Ligase ligiert. Die abermals notwendige Transformation und Plasmidisolierung erfolgte wie oben beschrieben.

Eine erfolgreiche Subklonierung ließ sich durch ein charakteristisches Restriktionsmuster nach Doppelverdau mit Eco RI und Bam HI im Agarose-Gel nachweisen.

Durch Sequenzierung des kompletten Trypsinogen-Gens konnte sichergestellt werden, dass nur die gewünschten Mutationen enthalten waren.

### Hefetranformation und Selektion

Der verwendete Hefezellstamm trägt die Bezeichnung *Saccharomyces cerevisiae DLM 101α [Mat a,leu 2-3,-112 his 2, 3-11,-15 can 1, ura 3△, pep4△, [cir^{o}], DM 23J.* Für die Herstellung kompetenter Hefezellen und die Transformation der pYT- Plasmide kam der EZ-Hefetransformationskit (ZYMO-RESEARCH) zum Einsatz. Die Selektion erfolgte auf Uracil-defizienten SC-Platten durch Bebrütung bei 30 °C für 3 bis 4 Tage. Einzelkolonien wurden weiterüberimpft auf Leucin-defiziente SC-Platten und ebenfalls 3 bis 4 Tage bei 30 °C inkubiert, wodurch die Kopienanzahl des Plasmides in den Zellen zunahm. Einzelkolonien dieser Platten wurden zum Animpfen der Vorkulturen des Leucin-defizienten SC-Flüssigmediums mit 8% Glukose herangezogen. Die Inkubation erfolgte unter schütteln bei 30 °C und 120 rpm für 3 Tage. Als Inokulum zum Animpfen der 1 Liter Hauptkulturen mit YPD- Medium (1 % Glucose, 1 % Bactopepton, 0,5% Hefeextrakt) wurden 20 ml Vorkultur eingesetzt. Die Inkubationsparameter entsprachen denen der Vorkultur, wobei nach 4 Tagen geerntet wurde.

### Isolierung und Reinigung der Trypsinvarianten

Durch Zentrifugation für 20 min bei 4000 rpm wurden zunächst die Zellen separiert und der auf pH 4,0 eingestellte Überstand erneut bei 12.000 rpm zentrifugiert. Der praktisch partikelfreie Trypsinogen-haltige Überstand wurde auf eine mit 2 mM Natriumacetat/100 mM Essigsäure (pH 4,5) equilibrierte Toyopearl 650 M (SUPELCO) Kationenaustauschersäule aufgetragen. Eluiert wurde mittels eines linearen pH-Gradienten beginnend von 2 mM Natriumacetat/ 100 mM Essigsäure (pH 4,5) bis 200 mM Tris/HCI (pH 8,0).

Durch SDS-Polyacrylamid-Gelelektrophorese unter Verwendung eines 15%-igen Polyacrylamid-Geles konnten die Trypsinogen enthaltenden Fraktionen ermittelt und zusammengefasst werden. Das Volumen der Proteinlösungen wurde mit Hilfe von Centriprep-Konzentratoren (AMICON) auf etwa 10 bis 15 ml eingeengt.

Die Aktivierung der Trypsinogen-Variante zum entsprechenden Trypsin D189K+K60E erfolgte mittels hochaufgereinigter Enterokinase (BIOZYME) bei pH 6,5 und wurde durch SDS-Gelelktrophorese kontrolliert.

Unter Verwendung eines Biocad Sprint Perfusionschrommatographie-Systems (PERSEPTIVE BIOSYSTEMS) wurde die Aufreinigung des aktivierten Enzyms durchgeführt. Die Auftrennung der Proteinproben erfolgte auf einer mit 5%-igen Bis-/Tris-Propan pH 6,0 equilibrierten POROS 20 HQ - Anionenaustauscher-Säule (4 x 100 mm, PERSEPTIVE BIOSYSTEMS) und nachfolgender Gradientenelution bis 95% 3M NaCl-Lösung. Die Trypsin-enthaltenden Fraktionen wurden mit Hilfe eines SDS-Geles auf Reinheit überprüft und zusammengefasst. Abschließend erfolgte die Dialyse gegen 1 mM HCI bei 4°C und Einengung der Proben mit Centriprep-Konzentratoren auf 2 bis 4 ml.

Die Endausbeuten beliefen sich auf etwa 2 bis 5 mg Protein pro Liter Kulturmedium.

### Konzentrationsbestimmung

Die Proteinkonzentration der Präparate wurde nach der Methode von Bradford an einem Spektrophotometer bei einer Wellenlänge von 595 nm bestimmt. Die Aufnahme der Eichkurve erfolgte anhand einer Rindertrypsin-Verdünnungsreihe zwischen 50 µm/ml und 1 mg/ml.

### Beispiel 2 - Synthese von Ht31 (493-515) mittels der Trypsinvariante D189K,K60E

Die Synthese des Zielmoleküls H-Asp-Leu-Ile-Glu-Glu-Ala-Ala-Ser-Arg-lle-Val-Asp-Ala-Val-Ile-Glu-Gln-Val-Lys-Ala-Ala-Gly-Ala-Tyr-OH (Ht31 (493-515)) erfolgte durch die Ligation des Oktapeptid-4-Guanidinophenylesters Boc-Asp-Leu-lle-Glu-Glu-Ala-Ala-Ser-OGp (2,2 mg, ca. 0,001 mmol) eingesetzt als Carboxykomponente mit dem als Aminokomponente fungierenden Peptid H-Arg-lie-Val-Asp-Ala-Val-lle-Giu-Gln-Val-Lys-Ala-Ala-Gly-Ala-Tyr-OH (1 mg, ca. 0,0005 mmol). Als Lösungsmittel diente ein wässriges Puffersystem mit einem 40%igen Anteil an organischem Lösungsmittel. Nach Vorlegen beider Reaktanden wurde die Reaktion durch Zugabe der Trypsin-Variante D189K,K60E gestartet und nach vollständigem Umsatz der Carboxykomponente analysiert. Die Enzymkatalyse führte zu einer vollständigen Acylierung der Aminokomponente. Die Identität des Syntheseproduktes wurde durch die üblichen Methoden der Organischen Chemie inklusive Aminosäureanalyse überprüft. Die Reaktion führte weder zu einer Modifizierung trifunktioneller Aminosäureseitenketten noch zu einer detektierbaren proteolytischen Spaltung der Reaktanden bzw. des Ligationsproduktes.

## Patentansprüche

1. Trypsinvariante D189K, K60E.

2. Verwendung der Trypsinvariante gemäß Anspruch 1 zur Synthese von Peptiden, Peptidmimetika oder Proteinen.

3. Verwendung der Trypsinvariante nach Anspruch 2, **dadurch gekennzeichnet, dass** zur Synthese des Zielmoleküls H-Asp-Leu-lle-Glu-Glu-Ala-Ala-Ser-Arg-Ile Val-Asp-Ala-Val-Ile-Glu-Gln-Val-Lys-Ala-Ala-Tyr-OH (Ht31 (493-515)) die Carboxykomponente Boc-Asp-Leu-Ile-Glu-Glu-Ala-Ala-Ser-OGp mit dem als Aminokomponenten fungierenden Peptid H-Arg-Ile-Val-Asp-Ala-Val-Ile-Glu-Gln-Val-Lys-Ala-Ala-Gly-Ala-Tyr-OH in einem wäßrigen Puffersystem mit 40 % Anteil an organischem Lösungsmittel durch Zugabe der Trypsinvariante D189K, K60E gestartet wird, was zur vollständigen Acylierung der Aminokomponente führt.

4. Verfahren zur Herstellung der Trypsinvariante D 189K, K60E durch einführen der gewünschten Mutation in einen Vektor mit Trypsin- oder Trypsinogen-codierender Sequenz, Transformation des neuen Vektors in einer geeigneten Wirtszelle insbesondere *E. coli*, Subklonierung der modifizierten Trypsin- oder Trypsinogensequenz in einen geeigneten Expressionsvektor, Expression der Trypsinvariante und gegebenenfalls Aktivierung der Trypsinogenvariante und anschließender Aufreinigung des Enzyms.

## Claims

1. Trypsin variant D 189K,K60E.

2. Use of the trypsin variant according to claim 1 to synthesize peptides, peptide mimetics or proteins.

3. Use of the trypsin variant according to claim 2, **characterized in that** the carboxy component Boc-Asp-Leu-Ile-Glu-Glu-Ala-Ala-Ser-OGp and the peptide H-Arg-Ile-Val-Asp-Ala-Val-Ile-Glu-Gln-Val-Lys-Ala-Ala-Gly-Ala-Tyr-OH acting as an amino component are used to synthesize the target molecule H-Asp-Leu-Ile-Glu-Glu-Ala-Ala-Ser-Arg-Ile-Val-Asp-Ala-Val-Ile-Glu-Gln-Val-Lys-Ala-Ala-Tyr-OH (Ht31 (493-515)) in an aqueous buffer system containing 40 % organic solvent, the synthesis being started by adding the trypsin variant D 189K,K60E which leads to the complete acylation of the amino component.

4. Method for the production of the trypsin variant D 189K,K60E by introducing the desired mutation into a vector containing a trypsin-coding or trypsinogen-coding sequence, transforming the new vector in a suitable host cell and in particular E. coli, subcloning the modified trypsin or trypsinogen sequence in a suitable expression vector, expressing the trypsin variant and optionally activating the trypsinogen variant and subsequently purifying the enzyme.

## Revendications

1. Variante de trypsine D189K, K60E.

2. Utilisation de la variante de trypsine selon la revendication 1 pour la synthèse de peptides, peptides mimétiques ou protéines.

3. Utilisation de la variante de trypsine selon la revendication 2, **caractérisée en ce que** pour la synthèse de la molécule cible H-Asp-Leu-Ile-Glu-Glu-Ala-Ala-Ser-Arg-Ile Val-Asp-Ala-Val-Ile-Glu-Gln-Val-Lys-Ala-Ala-Tyr-OH (Ht31 (493-515)), le composant carboxy Boc-Asp-Leu-Ile-Glu-Glu-Ala-Ala-Ser-OGp est amorcé avec le peptide H-Arg-lle-Val-Asp-Ala-Val-Ile-Glu-Gln-Val-Lys-Ala-Ala-Gly-Ala-Tyr-OH fonctionnant en tant que composant amino, dans un système aqueux tampon ayant une proportion de 40% de solvant organique, en ajoutant la variante de trypsine D189K, K60E, ce qui conduit à l'acylation complète du composant amino.

4. Procédé de préparation de la variante de trypsine D189K, K60E par introduction de la mutation souhaitée dans un vecteur avec une séquence codant pour la trypsine ou le trypsinogène, transformation du nouveau vecteur dans une cellule hôte appropriée, en particulier E. coli., sous-clonage de la séquence de trypsine ou de trypsinogène modifiée dans un vecteur d'expression approprié, expression de la variante de trypsine et éventuellement activation de la variante de trypsinogène suivie par la purification de l'enzyme.


### SEQUENZPROTOKOLL

<110> Roche Diagnostics GmbH und F. Hoffmann-La Roche AG
<120> Verfahren zur Synthese von Peptiden, Peptidmimetika und Proteinen
<130> 20978WO-SZ
<140> PCT/EP01/11500
   <141> 2001-10-05
<160> 7
<170> PatentIn Ver. 2.1
<210> 1
   <211> 21
   <212> DNA
   <213> Künstliche Sequenz
<220>
   <223> Beschreibung der künstlichen Sequenz:
   synthetischer Primer zur Erzeugung künstlicher Mutationen im Gen für anionisches Rattentrypsin
<400> 1
   ggaggcaaga acgattcctg c 21
<210> 2
   <211> 21
   <212> DNA
   <213> Künstliche Sequenz
<220>
   <223> Beschreibung der künstlichen Sequenz:
   synthetischer Primer zur Erzeugung künstlicher Mutationen im Gen für anionisches Rattentrypsin
<400> 2
   gcaggaatcg ttcttgcctc c 21
<210> 3
   <211> 21
   <212> DNA
   <213> Künstliche Sequenz
<220>
   <223> Beschreibung der künstlichen Sequenz:
   synthetischer Primer zur Erzeugung künstlicher Mutationen im Gen für anionisches Rattentrypsin
<400> 3
   cactgctatg agtcccgcat c 21
<210> 4
   <211> 21
   <212> DNA
   <213> Künstliche Sequenz
<220>
   <223> Beschreibung der künstlichen Sequenz:
   synthetischer Primer zur Erzeugung künstlicher Mutationen im Gen für anionisches Rattentrypsin
<400> 4
   gatgcgggac tcatagcagt g 21
<210> 5
   <211> 24
   <212> PRT
   <213> Künstliche Sequenz
<220>
   <223> Beschreibung der künstlichen Sequenz:
   Fusionspeptid, erzeugt durch Ligation zweier Peptide durch Trypsinmutante
<400> 5
   Asp Leu Ile Glu Glu Ala Ala Ser Arg Ile Val Asp Ala Val Ile Glu
    1              5                   10                  15
   Gln Val Lys Ala Ala Gly Ala Tyr
               20
<210> 6
   <211> 8
   <212> PRT
   <213> Künstliche Sequenz
<220>
   <223> Beschreibung der künstlichen Sequenz: Teilpeptid, das mit weiterem Teilpeptid durch enzymat. Aktivität der Trypsinmutante ligiert wird
<400> 6
   Asp Leu Ile Glu Glu Ala Ala Ser
    1              5
<210> 7
   <211> 16
   <212> PRT
   <213> Künstliche Sequenz
<220>
   <223> Beschreibung der künstlichen Sequenz: Teilpeptid, das mit weiterem Teilpeptid durch enzymat. Aktivität der Trypsinmutante ligiert wird
<400> 7
   Arg Ile Val Asp Ala Val Ile Glu Gln Val Lys Ala Ala Gly Ala Tyr
    1              5                   10                  15